(19) Europäisches Patentamt
European Patent Office
Office européen des brevets

(11) **EP 3 702 832 B1**

(12) **EUROPÄISCHE PATENTSCHRIFT**

(45) Veröffentlichungstag und Bekanntmachung des Hinweises auf die Patenterteilung:
**02.06.2021 Patentblatt 2021/22**

(51) Int Cl.:
***G02C 13/00*** *(2006.01)*

(21) Anmeldenummer: **19219216.9**

(22) Anmeldetag: **08.06.2018**

(54) **VERFAHREN, VORRICHTUNGEN UND COMPUTERPROGRAMM ZUM BESTIMMEN EINES NAH-DURCHBLICKPUNKTES**

METHOD, DEVICES AND COMPUTER PROGRAM FOR DETERMINING A CLOSE-UP VIEWPOINT

PROCÉDÉ, DISPOSITIFS ET PROGRAMME INFORMATIQUE DE DÉTERMINATION D'UN POINT VISUEL PROCHE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.06.2017 EP 17174925**

(43) Veröffentlichungstag der Anmeldung:
**02.09.2020 Patentblatt 2020/36**

(62) Dokumentnummer(n) der früheren Anmeldung(en) nach Art. 76 EPÜ:
**18728655.4 / 3 635 478**

(73) Patentinhaber: **Carl Zeiss Vision International GmbH**
**73430 Aalen (DE)**

(72) Erfinder:
• **GAMPERLING, Michael**
**89340 Leipheim (DE)**
• **ALVAREZ DIEZ, Cristina**
**73447 Oberkochen (DE)**
• **GLASENAPP, Carsten**
**73447 Oberkochen (DE)**

(74) Vertreter: **Sticht, Andreas**
**Kraus & Weisert**
**Patentanwälte PartGmbB**
**Thomas-Wimmer-Ring 15**
**80539 München (DE)**

(56) Entgegenhaltungen:
**EP-A2- 1 038 495**     **WO-A1-2017/064060**
**DE-A1-102005 003 699**     **DE-A1-102011 009 646**
**DE-A1-102014 200 637**     **US-A1- 2016 011 437**

**Beschreibung**

Die vorliegende Anmeldung betrifft Verfahren, Vorrichtungen und entsprechende Computerprogramme zum Bestimmen von Nah-Durchblickpunkten von Brillengläsern.

Bei der Einpassung von Brillengläsern in Brillenfassungen werden Zentrierparameter benötigt, um die Lage des Brillenglases in der Brillenfassung angepasst an die jeweilige Person, für die die Brille gedacht ist, einzupassen. Derartige Zentrierparameter sind unter anderem in der DIN EN ISO 13666:2012 definiert. Zu diesen Zentrierparametern zählen Fern-Durchblickpunkte, welche angeben, wo eine Blickrichtung der Person beim Blick in die Ferne durch die Brillengläser hindurchgeht und Nah-Durchblickpunkte, welche angeben, wo eine Blickrichtung der Person bei einem Blick in die Nähe, z.B. beim Lesen, durch die Brillengläser hindurchgeht. Fern-Durchblickpunkt und Nah-Durchblickpunkt sind insbesondere in der DIN EN ISO 13666: 2012 unter 5.16 und 5.17 definiert. Der Nah-Durchblickpunkt wird bei Gleitsichtgläsern benötigt, welche je nach Blickrichtung eine Korrektur für Fern-Sehen und eine Korrektur für Nah-Sehen ermöglichen (vergleiche DIN EN ISO 13666: 2012, 8.3.5). Häufig werden derartige Gleitsichtgläser heute verkauft und in eine Brillenfassung eingebaut, ohne dass die Position der Nah-Durchblickpunkte bestimmt wird, was dazu führen kann, dass der Brillenträger Objekte in der Nähe schlecht sieht.

Um dieses Problem zu beheben, sind verschiedene Ansätze bekannt, um Nah-Durchblickpunkte zu bestimmen.

Die JP 2005-342186 A offenbart beispielsweise ein Verfahren, welches bewegliche Lichtquellen benutzt, die von einer Fernblickrichtung (horizontal von dem Kopf der zu untersuchenden Person) zu einer zu der Horizontalen geneigten Nahblickrichtung wandern. Dabei werden mehrere Bildaufnahmen erstellt. Hier sind die Blickrichtungen durch die beweglichen Lichtquellen vorgegeben.

Die US 2014/009737 A1 offenbart eine relativ komplexe Vorrichtung, um Nah- und Fernzentrierdaten zu bestimmen. Hier werden zwei Kameramodule benutzt, um Bilder einer zu untersuchenden Person für eine Fernblickrichtung und eine Nahblickrichtung aufzunehmen.

Hierzu kann Software auf einem Tabletcomputer installiert werden, und auf einem Bildschirm des Tabletcomputers können Elemente angezeigt werden, die von der zu untersuchenden Person zu betrachten sind. Zudem muss ein Maßstab am Kopf der zu untersuchenden Person angebracht werden.

Die US 2010/149486 A1 misst einen Unterschied einer Kopfvorneigung zwischen einer Fernblickrichtung und einer Leseblickrichtung, wobei die genaue Blickrichtung beim Lesen nicht bestimmt wird, was zu Ungenauigkeiten führen kann.

Die US 2003/123026 A1 bestimmt einen Nah-Pupillenabstand (PD, Pupillary Distance) auf Basis einer Frontaufnahme des Kopfes der zu untersuchenden Person. Die dort verwendete Vorrichtung weist einen kurzen Betrachtungsabstand auf, wodurch es zu einer Verdeckung der Pupillen durch Teile der Brillenfassung kommen kann.

Aus der DE 103 00 188 A1 ist ein Verfahren bekannt, bei welchem Nah-Zentrierparameter mittels Bildaufnahmen bestimmt werden, wobei hier nicht angegeben ist, wie die Bestimmung genau erfolgen soll.

Die meisten der oben beschriebenen Herangehensweisen haben den Nachteil, dass ein Referenzobjekt mit bekannten Abmessungen, beispielsweise ein sogenannter Messbügel, an der Brillenfassung oder am Gesicht angeordnet sein muss, um die Anordnung der Brillenfassung im Raum zu erkennen. Die Verwendung eines derartigen Messbügels stellt jedoch eine nicht unerhebliche Beeinflussung der Position der Fassung im Gesicht der Person dar, und die Person selbst kann durch das Vorhandensein des Messbügels beeinflusst werden, sodass hier Ungenauigkeiten bei der Messung entstehen können. Zudem werden die Nah-Durchblickpunkte bei diesen Verfahren teilweise nur indirekt bestimmt.

Die EP 2 913 704 A1 offenbart eine Vorrichtung, bei welchen Zentrierparameter für eine Fernblickrichtung und eine Nahblickrichtung bestimmt werden können. Die Fernblickrichtung und Nahblickrichtung sind dabei konstruktionsbedingt durch die Vorrichtung vorgegeben. Somit entspricht die durch die Vorrichtung vorgegebene Nahblickrichtung nicht unbedingt der tatsächlichen Nahblickrichtung, welche die zu untersuchende Person beispielsweise beim Lesen einnimmt.

Die WO 2014/061294 A1 offenbart ein Verfahren, bei welchem ein Kopf einer zu untersuchenden Person mit Stroboskoplicht beleuchtet wird und jeweils für eine Fernblickposition und eine Nahblickposition mit einer Kamera aufgenommen wird, welche zwischen Fernblickposition und Nahblickposition beweglich ist. Die durch das Stroboskoplicht erzeugten Reflexe der Pupillen werden dann ausgewertet. Auch hier entspricht die Nahblickposition nicht notwendigerweise der natürlichen Leseposition der Person, sondern ist durch die Kameraposition bestimmt. Zudem wird eine Stroboskopbeleuchtung benötigt.

Die FR 3 021 205 A1 offenbart eine Herangehensweise zur Bestimmung von Haltungsparametern, bei welchen eine Person bei verschiedenen Tätigkeiten, beispielsweise Lesen, aufgenommen wird, und relative Positionen des Kopfes in den Haltungen auf Basis von übereinstimmenden Punkten bestimmt werden. Eine Bestimmung von Zentrierparametern wie beispielsweise einem Nah-Durchblickpunkt wird in dieser Druckschrift nicht erwähnt.

Die 10 2011 009 646 A1 offenbart ein Verfahren zum Bestimmen eines Nah-Durchblickpunktes, bei dem ein Bild eines Kopfes einer Person, während die Person auf ein bewegliches Nahblickziel blickt, aufgenommen wird.

Die Position und/oder Orientierung des Nahblickziels wird bestimmt, und der Nah-Durchblickpunktes (51) wird auf Basis des Bildes und auf Basis der Position und/oder Orientierung des Nahblickziels bestimmt.

Die US 2002/0105530 A1 offenbart das Erzeugen eines 3D-Modells eines Gesichts mittels eines Kamerasystems und einer 3D-Modellierungsanwendung, wobei ein voll texturiertes 3D-Modell des Gesichts erzeugt wird.

Die EP 2 963 482 A1 offenbart ein System zum Linsendesign.

Die US 2014/0293219 A1 offenbart ein Verfahren zum Bestimmen des Leseabstands einer Person.

Die europäische Anmeldung EP 3 270 099 A1 offenbart ein Verfahren zur Bestimmung eines Hornhaut-Scheitelabstands auf Grundlage von zwei Seitenbildern eines Kopfes einer Person, wobei ein erstes Seitenbild mit aufgesetzter Brille und ein zweites Seitenbild ohne aufgesetzte Brille mit einer Kamera aufgenommen werden.

Die EP 1 038 495 A2 offenbart ein Verfahren zum Bestimmen eines Nah-Durchblickpunktes, bei dem ein Bild eines Kopfes einer Person mit einem in dem Nahblickziel eingebauten Kamera vorgenommen wird, während die Person auf das Nahblickziel blickt. Eine Blickrichtung der Person beim Blick auf das Nahblickziel wird auf Basis des Bildes bestimmt, wozu ein an der Brillenfassung befestigter Maßstab benutzt wird.

Die US 2016/011437 A1 offenbart ein System zum Entwerfen eines Brillenglases, welches eine Detektion eines Augendrehpunkts und ein Bild verwendet. Zudem kann eine Blickrichtung mittels einer RGB-D-Kamera basierend auf 2D- und Tiefeninformationen bestimmt werden.

Die DE 10 2011 009 646 A1 offenbart ein Verfahren zum Bestimmen eines Nah-Durchblickpunktes mit einer Kameravorrichtung und einem Nahblickziel, wobei das Nahblickziel eine bewegliche mechanische Verbindung mit einer Säule aufweist, wobei Position und/oder Orientierung des Nahblickziels im Raum mit einem Sensor erfasst wird.

Die WO 2017/064060 A1 offenbart ein Verfahren zum Entwerfen eines Brillenglases für Gleitsichtbrillen, bei dem eine Blickrichtung und insbesondere eine mittlere Blickrichtung bestimmt werden.

Die DE 10 2014 200 637 A1 offenbart ein Verfahren zum Bestimmen eines Anpassparameters für ein Brillenglas, wozu ebenfalls eine Bildaufnahme mit einer Kamera verwendet wird. Dabei wird ein Neigungssensor verwendet, um eine Neigung der Kamera zu bestimmen.

Die DE 10 2005 003 699 A1 offenbart eine weitere Vorrichtung zur Bestimmung von optischen Parametern eines Benutzers anhand von Bildaufnahmen.

Die DE 10 2014 200 637 A1 offenbart ein Verfahren gemäß dem Oberbegriff von Anspruch 1 und eine Vorrichtung gemäß dem Oberbegriff von Anspruch 13. Ausgehend von der DE 10 2014 200 637 A1 ist es eine zweite Aufgabe der vorliegenden Anmeldung, die Bestimmung des Nah-Durchblickpunktes ohne das Verwenden von Seitenzielmarken zu ermöglichen und das Verfahren robuster gegen Verdeckung der Augen durch einen Brillenbügel in dem Seitenbild zu machen.

Hierzu werden erfindungsgemäß ein Verfahren gemäß Anspruch 1 und eine Vorrichtung gemäß Anspruch 13 bereitgestellt.

Die Erfindung wird nachfolgend kurz diskutiert. Verwendete Begriffe werden teilweise erst nach dieser kurzen Diskussion definiert.

In einem nicht beanspruchten Erfindungsaspekt wird ein Verfahren zum Bestimmen eines Nah-Durchblickpunktes bereitgestellt. Das Verfahren umfasst:

Aufnehmen eines Bildes eines Kopfes einer Person, wobei das Aufnehmen des Bildes mit einer in dem Nahblickziel eingebauten Bildaufnahmeeinrichtung erfolgt, während die Person auf ein Nahblickziel blickt,

wobei das Bild eine Pupillenposition und/oder Corneaposition von Augen zeigt,

wobei das Nahblickziel beweglich ist, und wobei das Verfahren weiter umfasst:

Bestimmen einer Orientierung des Nahblickziels, und

Bestimmen einer Blickrichtung der Person beim Blick auf das Nahblickziel auf Basis des Bildes.

Das Verfahren ist dadurch gekennzeichnet, dass das Bestimmen des Nah-Durchblickpunktes auf Basis des Bildes und auf Basis der Blickrichtung und auf Basis der Position und/oder Orientierung des Nahblickziels erfolgt, wobei

das Bestimmen der Blickrichtung auf einer Lage der Pupillenposition und/oder Corneaposition der Augen relativ zu einer Symmetrieachse der Bildaufnahmeeinrichtung und einem Bildwinkel und einer Auflösung der Bildaufnahmeeinrichtung basiert, wobei die Richtung der Symmetrieachse durch die Orientierung des Nahblickziels bestimmt ist.

Ferner wird in dem nicht beanspruchten Erfindungsaspekt eine Vorrichtung zum Bestimmen eines Nah-Durchblickpunktes bereitgestellt. Die Vorrichtung umfasst:

ein Nahblickziel (40; 60), welches beweglich ist und eine Bildaufnahmeeinrichtung (62) und eine Positionserfassungseinrichtung (61) umfasst,

wobei die Bildaufnahmeeinrichtung (13; 62) zum Aufnehmen eines Bildes einer Person, während die Person auf ein Nahblickziel blickt, eingerichtet ist,

wobei die Positionserfassungseinrichtung (42; 61) zum Bestimmen einer Orientierung des Nahblickziels eingerichtet ist, und

eine Recheneinrichtung (14) zum Bestimmen des Nah-Durchblickpunktes.

Die Vorrichtung ist dadurch gekennzeichnet, dass die Recheneinrichtung derart eingerichtet ist, dass das Bestimmen des Nah-Durchblickpunktes auf Basis des Bildes und auf Basis der Blickrichtung und auf Basis der Position und/oder Orientierung des Nahblickziels erfolgt, wobei

das Bestimmen der Blickrichtung auf einer Lage der Pupillenposition der Augen relativ zu einer Symmetrieachse der Bildaufnahmeeinrichtung auf einem Bildwinkel und einer Auflösung der Bildaufnahmeeinrichtung basiert, wobei die Richtung der Symmetrieachse durch die Orientierung des Nahblickziels bestimmt ist.

Ein solches Verfahren und eine solche Vorrichtung hat gegenüber dem Verfahren der europäischen Patentanmeldung Nr. 1 038 495 A2 den Vorteil, dass eine Blickrichtung der Person bestimmt werden kann, ohne dass eine Notwendigkeit besteht, Messstäbe, die an dem Brillengestell oder an der Person angebracht werden müssen, zu verwenden, da hier die Blickrichtung auf Basis des aufgenommenen Bildes und Eigenschaften der Bildaufnahmeeinrichtung, nämlich Auflösung und Bildwinkel, erfolgt.

Eine Bestimmung auf Basis von oder basierend auf einer oder mehreren Größen (z.B. Bildwinkel und Auflösung) bedeutet, dass diese Größen in die Bestimmung eingehen. Konkrete Beispiele für die Bestimmung werden später erläutert.

Eine Bildaufnahmeeinrichtung ist dabei allgemein eine Einrichtung, mit der ein digitales Bild von einem Objekt, in diesem Fall einem Kopf einer Person oder eines Teils davon, erstellt werden kann. Eine derartige Bildaufnahmeeinrichtung kann eine oder mehrere Kameras umfassen.

Die Auflösung der Bildaufnahmeeinrichtung gibt an, wie viele Bildpunkte (auch als Pixel bezeichnet) ein so erzeugtes digitales Bild aufweist. Sie wird bei üblichen rechteckigen Bildern üblicherweise in L x H angegeben, wobei L und H die Anzahl der Bildpunkte entlang der Kanten des Bildes angeben. Ein solches rechteckiges Bild kann als Bildpunktmatrix mit Länge L und Breite H angesehen werden. Die Auflösung hängt dabei von einem verwendeten Bildsensor ab.

Unter Symmetrieachse der Kamera wird eine Achse der Kamera verstanden, bei der ein entlang der Symmetrieachse einfallender Lichtstrahl in der Mitte des Bildes abgebildet wird.. Bei einer Rotation der Kamera um die Symmetrieachse ändert sich die beobachtete Richtung von aufgenommenen Objekten, gemessen als Winkel relativ zur Symmetrieachse, nicht. Bei vielen Bildaufnahmeeinrichtungen entspricht diese Symmetrieachse der optischen Achse eines Objektivs der Bildaufnahmeeinrichtung.

Der Bildwinkel gibt an, in welchem Winkelbereich gesehen von der Bildaufnahmeeinrichtung Objekte in dem Bild erfasst werden und entspricht der üblichen Verwendung dieses Begriffs in der Fotografie, siehe Beispielsweise    https://de.wikipedia.org/wiki/Bildwinkel; Stand 6.5.2018. Der Bildwinkel wird ebenfalls von einem verwendeten Objektiv bestimmt.

Durch Auflösung und Bildwinkel besteht eine feste Beziehung zwischen einem Winkel, unter dem ein Objekt zu der Symmetrieachse der Bildaufnahmeeinrichtung gesehen von der Bildaufnahmeeinrichtung steht, und dem Bildpunkt oder den Bildpunkten, bei denen das Objekt in dem Bild erscheint. Indem also z.B. eine Lage eines Auges oder Teils hiervon in dem Bild identifiziert wird, kann die Blickrichtung relativ zur Symmetrieachse bestimmt werden.

Erfindungsgemäß wird ein Verfahren zum Bestimmen eines Nah-Durchblickpunktes bereitgestellt. Das Verfahren umfasst:

Aufnehmen eines Frontbildes und eines ersten Seitenbildes eines Kopfes einer Person, während die Person eine Brillenfassung trägt und auf ein Nahblickziel blickt, wobei das Nahblickziel beweglich ist, Bestimmen einer Position und Orientierung des Nahblickziels, und Bestimmen einer Lage der Brillenfassung basierend auf dem Frontbild und dem Seitenbild,

Bestimmen einer Blickrichtung der Person beim Blick auf das Nahblickziel, und Bestimmen des Nah-Durchblickpunktes der Brillenfassung auf Basis der Blickrichtung und der Lage der Brillenfassung.

Das Verfahren ist dadurch gekennzeichnet, dass das Bestimmen der Blickrichtung umfasst: Erzeugen eines zweiten Seitenbilds des Kopfes basierend auf einem 3D-Modell des Kopfes,

Bestimmen der Position einer Pupille und/oder einer Cornea eines Auges der Person basierend auf dem ersten Seitenbild und dem zweiten Seitenbild, Berechnen der Blickrichtung als Differenz zwischen der Position des Nahblickziels und der Position der Pupille oder der Cornea und dem ersten Bild.

Im Gegensatz zur eingangs erwähnten EP 3 270 099 A1 wird das zweite Seitenbild hier also aus einem 3D-Modell erzeugt und nicht mittels einer Kamera aufgenommen.

Ferner wird erfindungsgemäß eine Vorrichtung zum Bestimmen eines Nah-Durchblickpunktes (51) bereitgestellt. Die Vorrichtung umfasst:

Ein Nahblickziel (40; 60), wobei das Nahblickziel (40; 60) beweglich ist, eine Bildaufnahmeeinrichtung (13; 62) zum Aufnehmen eines Frontbildes und eines ersten Seitenbildes eines Kopfes einer Person, während die Person auf das Nahblickziel blickt, eine Positionserfassungseinrichtung (42; 61) zum Bestimmen einer Position und Orientierung des Nahblickziels, eine Recheneinrichtung (14) zum Bestimmen des Nah-Durchblickpunktes.

Die Vorrichtung ist dadurch gekennzeichnet, dass die Recheneinrichtung (14) eingerichtet ist zum:

> Erzeugen eines ersten Seitenbilds des Kopfes basierend auf einem 3D-Modell des Kopfes,
> Bestimmen der Position einer Pupille und/oder einer Cornea eines Auges der Person basierend auf dem ersten Seitenbild und dem zweiten Seitenbild,
> Berechnen der Blickrichtung als Differenz zwischen der Position des Nahblickziels und der Position der Pupille oder der Cornea und dem ersten Bild.

Ein solches Verfahren und eine solche Vorrichtung haben gegenüber dem Verfahren der DE 10 2014 200 637 A1 den Vorteil, dass die Bestimmung des Nah-Durchblickpunktes ohne das Verwenden von Seitenzielmarken ermöglicht wird und dass das Verfahren auch in solchen Fällen erfolgreich angewendet werden kann, wenn die Augen der Person auf dem Seitenbild durch einen Brillenbügel verdeckt sind.

Unter Differenz wird eine Subtraktion vektorieller Größen verstanden.

Bevorzugt ist das erfindungsgemäße Verfahren dadurch gekennzeichnet, dass das Bestimmen der Position der Pupille und/oder der Cornea basierend auf dem ersten Seitenbild und dem zweiten Seitenbild ein zur Deckung bringen des ersten Seitenbildes mit dem zweiten Seitenbild umfasst. Durch das zur Deckung bringen können Bildbereiche des ersten Seitenbildes und des zweiten Seitenbildes hinsichtlich ihrer Lage einfach miteinander identifiziert werden.

Bevorzugt ist das Verfahren zusätzlich oder alternativ dadurch gekennzeichnet, dass das Bestimmen der Position der Pupille und/oder der Cornea basierend auf dem ersten Seitenbild und dem zweiten Seitenbild ein Ersetzen eines Teils des ersten Seitenbildes durch einen entsprechenden Teil des zweiten Seitenbildes, der die Pupille und/oder Cornea beinhaltet, umfasst. So kann die Lage von Pupille und/oder Cornea in dem ersten Seitenbild kenntlich gemacht werden, auch wenn Pupille und/oder Cornea im ersten Seitenbild zunächst durch einen Brillenbügel verdeckt sind.

Unter Ersetzen eines Teils des ersten Seitenbildes wird dabei verstanden, dass ortsselektiv Informationen des ersten Seitenbildes durch Informationen des zweiten Seitenbildes ersetzt werden. Beispielsweise können Seitenbilder als Bildpunktmatritzen in zwei Dimensionen mit zwei Indizes x und y dargestellt werden. Ein Ersetzen eines Teils kann hierbei bedeuten, dass einzelne Bildpunkt-Werte für bestimmte x- und y- Werte xi und yi des ersten Seitenbildes durch Rastermatrix-Werte des zweiten Seitenbildes für gleich x und y Werte xi und yi ersetzt werden. Das erste und das zweite Seitenbild können insbesondere auf Anzeigen, beispielsweise einem Bildschirm, zur Betrachtung durch einen Optiker, dargestellt werden.

Die erfindungsgemäße Vorrichtung kann bevorzugt entsprechend den obigen bevorzugten Ausführungsformen des Verfahrens eingerichtet sein.

Eine derartige Herangehensweise, bei der die Position von Pupille und/oder Cornea durch das erste Seitenbild und das zweite Seitenbild bestimmt wird, kann auch bei dem ersten Erfindungsaspekt angewendet werden. Dabei kann die Bestimmung der Blickrichtung zudem auf der so bestimmten Position von Pupille und/oder Cornea erfolgen. Insbesondere kann von der Position von Pupille und/oder Cornea auf eine Position des Augendrehpunktes rückgeschlossen werden, wie später näher erläutert werden wird, und die Blickrichtung kann dann so bestimmt werden, dass sie am Augendrehpunkt "ansetzt", also vom Augendrehpunkt ausgehend verläuft.

In dem dritten nicht beanspruchten Erfindungsaspekt wird ein Verfahren zum Bestimmen eines Nah-Durchblickpunktes bereitgestellt. Das Verfahren umfasst:

Aufnehmen eines Bildes eines Kopfes einer Person, während die Person auf ein Nahblickziel blickt, wobei das Nahblickziel beweglich ist.

Das Verfahren ist dadurch gekennzeichnet, dass das Nahblickziel eine bewegliche mechanische Verbindung mit einer Säule aufweist, die mindestens einen Sensor umfasst und dass das Verfahren weiter umfasst:

> Bestimmen einer Position und/oder Orientierung des Nahblickziels (40) im Raum mittels des mindestens einen Sensors, und
> Bestimmen des Nah-Durchblickpunktes auf Basis des Bildes und auf Basis der Position und/oder Orientierung des Nahblickziels .

Ferner wird in dem nicht beanspruchten dritten Erfindungsaspekt eine Vorrichtung zum Bestimmen eines Nah-Durchblickpunktes bereitgestellt. Die Vorrichtung umfasst:

> ein Nahblickziel , wobei das Nahblickziel beweglich ist, eine Bildaufnahmeeinrichtung (13; 62) zum Aufnehmen eines Bildes eines Kopfes einer Person, während die Person auf das Nahblickziel blickt, und eine Recheneinrichtung (14) zum Bestimmen des Nah-Durchblickpunktes.

Die Vorrichtung ist dadurch gekennzeichnet, dass das Nahblickziel (40) eine bewegliche mechanische Verbindung (42) mit einer Säule (12) aufweist, die mindestens einen Sensor umfasst, der eingerichtet ist, einer Position und Orientierung des Nahblickziels zu bestimmen, und an die Recheneinrichtung (14) bereitzustellen.

Ein solches Verfahren und eine solche Vorrichtung hat gegenüber dem Verfahren der japanische Patentpublikation Nr. 2003329541 A den Vorteil, dass die Bestimmung des Nah-Durchblickpunktes ohne das Verwenden von Seitenzielmarken ermöglicht wird und dass die Person das Nahblickziel über einen großen Bereich bewegen kann, um eine angenehme Lesehaltung zu erreichen.

Bei den obigen Verfahren und Vorrichtungen des ersten und dritten Aspekts können die angesprochenen Bilder aufgenommen werden, während die Person eine Brillenfassung trägt, oder aufgenommen werden, wenn die Person keine Brillenfassung trägt. Wie später näher erläutert werden wird kann im ersten Fall dann eine Lage der Brillenfassung aus den Bildern bestimmt werden, während im letzteren Fall die Lage der Brillenfassung virtuell anhand eines Modells bestimmbar ist.

Zu beachten ist, dass der Bezug auf "einen Nah-Durchblickpunkt" hier bedeutet, dass der Nah-Durchblickpunkt für ein Auge oder für beide Augen der Person bestimmt werden können.

Unter der Position des Nahblickziels ist die Position im dreidimensionalen Raum zu verstehen, wie sie beispielsweise in kartesischen Koordinaten bezüglich eines raumfesten Koordinatensystems angegeben werden kann. Die Orientierung des Nahblickziels beschreibt, wie das Nahblickziel an seiner Position ausgerichtet ist, beispielsweise in welche Richtung eine Seite des Nahblickziels zeigt, auf welche die Person zur Messung blicken soll. Die Kombination von Position und Orientierung wird nach der DIN EN ISO 8373: 2012-03 auch als Pose bezeichnet und spielt insbesondere auch in der Robotertechnik eine große Rolle.

Ein Nahblickziel ist ein Gegenstand, auf welches die Person zur Bestimmung des Nah-Durchblickpunktes blicken soll, wobei beispielweise eine Lesehaltung eingenommen werden kann. Hierzu kann das Nahblickziel einen zu lesenden Text oder ein zu betrachtendes Bild aufweisen, welches die Person in einer normalen Lesehaltung betrachten soll. Dadurch, dass das Nahblickziel beweglich ist, kann eine natürliche Lesehaltung eingenommen werden, und die Messung ist nicht auf eine von der Vorrichtung vorgegebene Lesehaltung beschränkt. Durch die Bestimmung auf Basis des Bildes und der Position und/oder Orientierung des Nahblickziels kann zudem auf die Verwendung eines Messbügels oder ähnlichen Gegenstands verzichtet werden.

Bevorzugt umfasst das Bestimmen des Nah-Durchblickpunktes ein Bestimmen einer Blickrichtung der Person beim Blicken auf das Nahblickziel auf Basis des Bilds umfasst, wobei das Bestimmen des Nah-Durchblickpunktes auf Basis der Blickrichtung erfolgt. Mittels der Blickrichtung kann der Nah-Durchblickpunkt auf einfache Weise ermittelt werden

Hierzu umfasst das Verfahren bevorzugt weiterhin ein Bestimmen einer relativen Lage einer von der Person getragenen Brillenfassung zu einem Augendrehpunkt der Person.

So kann der Nah-Durchblickpunkt einfach bestimmt werden, indem ausgehend von dem Augendrehpunkt ein Schnittpunkt der Blickrichtung mit einem Brillenglas, dessen Lage durch die Lage der Brillenfassung definiert ist, ermittelt wird. Zudem wird so lediglich die Blickrichtung benötigt, jedoch nicht der Abstand zwischen der Person und dem Nahblickziel, der beliebig gewählt werden kann. Unter einer Brillenfassung ist dabei in Übereinstimmung mit DIN EN ISO 7998 und DIN EN ISO 8624 ein Gestell oder eine Halterung zu verstehen, mittels dem/der Brillengläser am Kopf getragen werden können. Der Begriff wie hier verwendet beinhaltet insbesondere auch randlose Brillenfassungen.

Das Bestimmen der relativen Lage der von der Person getragenen Brillenfassung zu dem Augendrehpunkt der Person kann mittels weiterer oder der Bildaufnahmen erfolgen, beispielsweise im Zuge von Bildaufnahmen, welche zur Bestimmung des Fern-Durchblickpunktes erstellt werden, bei denen die Person die Brillenfassung trägt. Entsprechende Vorrichtungen, welche Zentrierparameter wie den Fern-Durchblickpunkt auf Basis mehrere Bildaufnahmen bestimmen, sind in den europäischen Patentanmeldungen Nr. 17 153 559.4 sowie Nr. 17 153 556.0 beschrieben.

So kann mithilfe von Parametern wie der relativen Lage der von der Person getragenen Brillenfassung zu dem Augendrehpunkt der Person, die von einer vorherigen Bestimmung des Fern-Durchblickpunktes ohnehin vorliegen, ein Nah-Durchblickpunkt einfach bestimmt werden.

Aus Fern- und Nah-Durchblickpunkt kann dann zusätzlich die sogenannte Progressionskanallänge (siehe 14.1.25 in DIN EN ISO 13666:2013-10) bestimmt werden, die den Abstand zwischen Nah-Durchblickpunkt und Fern-Durchblickpunkt angibt. Zudem oder alternativ kann der nasale Abstand der Nah-Durchblickpunkte relativ zu den Fern-Durchblickpunkten bestimmt werden. Dieser nasale Abstand wird manchmal als "Inset" bezeichnet, stimmt aber nicht notwendigerweise mit dem zwischenzeitlich in der DIN EN ISO 13666:2013-10 unter 14.2.13 normierten Inset überein. Herkömmlicherweise ist dieser nasale Abstand durch das Glasdesign des Gleitsicht-Brillenglases oder des Bifokal-Brillenglases fest vorgegeben. Dadurch wird nicht berücksichtigt, dass dieser nasale Abstand beider Seiten ggf. asymmetrisch sein kann, z.B. dann, wenn der Proband den Kopf normalerweise leicht seitlich geneigt hält, der Blick zum Lesen aber senkrecht nach unten schwenkt, ohne die Seitenneigung des Kopfes zu verändern. Eine derartige Asymmetrie kann durch die Bestimmung dieses nasalen Abstands auf Basis der wie oben beschrieben bestimmten Nah-Durchblickpunkte und Fern-Durchblickpunkte ermittelt werden.

Bei einer Variante erfolgen dabei das Aufnehmen des Bildes als Aufnahme mehrerer Bilder mit der gleichen Kameraeinrichtung, mittels derer die weiteren Bildaufnahmen erstellt werden. Die in den oben genannten europäischen Patentanmeldungen Nr. 17 153 5594 sowie Nr. 17 153 556.0 beschriebene Kameraeinrichtung weist hierfür mehrere Kameras auf, welche in einem Winkel von etwa 90° zueinander angeordnet sind, um Front- und Seitenaufnahmen zu erstellen. In so aufgenommenen mehreren Bildern können dann mittels der in den obigen Anmeldungen beschriebenen Algorithmen eine Lage der Brillenfassung und eine Augenposition bestimmt werden. Diese Bestimmung kann auf Basis einer Bestimmung der Lage der Brillenfassung in den weiteren Bildaufnahmen vorgenommen werden, d. h. die dort bestimmte Form der Brillenfassung kann mittels Bildanalyse-Algorithmen wie beispielsweise in der europäischen Patentanmeldung Nr. 17153651.9 beschrieben in dem aufgenommenen Bild bestimmt werden, oder es kann eine gemeinsame Bestimmung durchgeführt werden. Bei den in der europäischen Patentanmeldung Nr. 17153651.9 beschriebenen Algorithmen werden insbesondere Lagen von Glasrändern von Brillengläsern detektiert, welche Fassungsrändern der Brillenfassung entsprechen. Hierdurch ist die Lage der Brillenfassung, insbesondere die Lage der Fassungsebene (DIN EN ISO 13666:2012; 17.2), ebenfalls bekannt. Eine Bestimmung einander entsprechender Elemente, in diesem Fall der Brillenfassungen, in den mehreren Bildern sowie in den weiteren Bildaufnahmen kann auch wie in der US 6,944,320 B2 oder der US 7,149,330 B2 beschrieben erfolgen. Auf diese Weise ist eine robuste Bestimmung der Lage der Brillenfassung möglich.

Mit der Lage der Brillenfassung, insbesondere der Fassungsebene, kann dann wie oben beschrieben der Nah-Durchblickpunkt bestimmt werden.

Alternativ kann die Bestimmung der Lage der Brillenfassung auch gemeinsam in dem aufgenommenen Bild und den weitere Bildaufnahmen erfolgen. Eine gemeinsame Verarbeitung des Bilds und der weiteren Bildaufnahme zur Bestimmung der Brillenfassungen, insbesondere der Fassungsränder und somit der Brillengläser, erhöht die Robustheit des Verfahrens weiter.

Bei einer derartigen Herangehensweise mit einer Kameraeinrichtung kann die Position des Nahblickziels im Raum beispielsweise durch Objektverfolgung mittels weiterer Kameras oder durch eine mechanische mit Sensoren versehene Verbindung zu der Kameravorrichtung erfasst werden. Eine solche Objektverfolgung ist z.B. in T. Dedek, "Entwurf und Implementierung einer Objektverfolgung unter Verwendung einer Smart-Camera mit PTZ-Funktionalität", Bachelorarbeit 2009, https://www.ipi.uni-hannover.de/fileadmin/institut/pdf/Abschlussarbeiten/Bachelorarbeit_Dedek_2009.pdf, beschrieben.

Die Blickrichtung kann in diesem Fall bestimmt werden, indem auf Basis des Bildes Pupillenpositionen in drei Dimensionen bestimmt werden und die Blickrichtung dann als Differenz zwischen der Position des Nahblickziels und der Position der Pupillen bestimmt werden. Die Position der Pupillen kann dabei mit den in den oben genannten Anmeldungen bestimmten Verfahren bestimmt werden.

Der oben erwähnte Augendrehpunkt, Englisch: "Center of Rotation" (COR), kann mithilfe bekannter empirischer Daten (siehe beispielsweise Bennett, A., Rabbetts, R.: Clinical Visual Optics, Third Edition, Oxford (1998), S143/144) oder unter Berücksichtung einer refraktionsabhängigen Lage hinter der Pupille bestimmt werden und somit dessen Position relativ zu der Lage der Brillenfassung und somit den Fassungsrändern angegeben werden. Auf diese Weise ist die Bestimmung mittels herkömmlicher Verfahren möglich.

Bei einer anderen Ausführungsform erfolgt das Aufnehmen des Bildes durch eine in das Nahblickziel eingebaute Kamera, und die Orientierung des Nahblickziels und somit der Kamera wird durch Neigungssensoren in dem Nahblickziel bestimmt. Die Pupillenposition der Augen wird dann in dem aufgenommenen Bild bestimmt. Bei bekanntem Bildwinkel und Auflösung der Kamera, welche sich aus den Spezifikationen der Kamera ergeben, kann wie oben für den ersten Aspekt angegeben für die Pupillen eine Richtung relativ zu einer Symmetrieachse der Kamera (die üblicherweise der optischen Achse der Kamera entspricht) angegeben werden. Die Richtung dieser Symmetrieachse ergibt sich aus der ermittelten Neigung des Nahblickziels. Somit kann bei dieser Ausführungsform die Blickrichtung beider Augen direkt bestimmt werden, und wie oben erläutert auf Basis der Blickrichtung auf das Nahblickziel ein Nah-Durchblickpunkt für jedes Auge.

Die Richtung der Pupillen kann hierbei angegeben werden, wenn die Person direkt in die Kamera blickt. In anderen Ausführungsbeispielen kann das Nahblickziel einen Anzeigebereich umfassen. Unter Anzeigebereich wird hierbei ein Bereich verstanden, auf dem Informationen angezeigt werden können, beispielsweise ein Bildschirm. Informationen können hierbei Bilder, Zeichnungen, Text oder Sehzeichen, beispielsweise Landolt-Ringe sein. Bei einem Tabletcomputer oder Smartphone ist der Anzeigebereich beispielsweise das sogenannte Display des Tablets oder Smartphones.

Der Anzeigebereich kann in einem Abstand zur Kamera angeordnet sein. In den Fällen, bei denen die Person auf den Anzeigebereich blickt, kann das Bestimmen der Blickrichtung zusätzlich auf einem Abstand der Kamera des Nahblickziels von dem Anzeigebereich oder zu einem Teil des Anzeigebereichs, auf dem Informationen angezeigt werden, basieren. Hierdurch kann die Genauigkeit des Verfahrens weiter gesteigert werden, so berücksichtigt werden kann, dass sich durch den Blick auf den Anzeigebereich, der von der Kamera beabstandet ist, die tatsächliche Blickrichtung von der Blickrichtung, die wie oben beschrieben nur auf Basis von Bildwinkel und Auflösung der Kamera bestimmt wird, unterscheidet.

Dabei kann bei einer solchen Korrektur, die berücksichtigt, dass die Person auf den Anzeigebereich und nicht direkt auf die Kamera blickt, der Abstand des Nahblickziels von der Person bei dem Bestimmen der Blickrichtung berücksichtigt werden. Diese Korrekturen beruhen dann auf einfachen geometrischen Überlegungen. Bei anderen Ausführungsbeispielen kann die Person auf die Kamera blicken, d.h. die Kamera dient selbst als Nahblickziel.

Die Kamera des Nahblickziels kann intrinsisch kalibriert sein, um Bildfeldverzeichnungen zu korrigieren. Intrinsische Kalibrierung bedeutet, dass die Bildfeldverzeichnung gemessen wird und dann korrigiert wird. Dies erhöht die Genauigkeit der Bestimmung des Nah-Durchblickpunktes. Falls die Genauigkeit der Bestimmung auch ohne eine solche Kalibrierung ausreichend ist, beispielsweise weil die Verzeichnungen kleiner als 10 % sind, kann die intrinsische Kalibrierung der Kamera des Nahblickziels auch entfallen.

Bevorzugt wird in einem derartigen Fall als Nahblickziel ein Tablet-Computer oder Smartphone verwendet. Derartige Geräte verfügen mit der Frontkamera über eine geeignete Kamera, weisen eingebaute Neigungssensoren und eine Anzeige zum Anzeigen beispielsweise von einem zu lesenden Text oder eines zu betrachtenden Bildes auf, sodass hier durch einfache Programmierung das erfindungsgemäße Verfahren implementiert werden kann. Das aufgenommene Bild und die gemessenen Neigungen können dann direkt in dem Tablet oder Smartphone ausgewertet werden oder mittels einer Schnittstelle, wie sie ebenfalls üblicherweise vorhanden ist, drahtgebunden oder drahtlos an eine Recheneinrichtung zur weiteren Auswertung übersendet werden.

Das Verfahren kann zudem eine Korrektur der bestimmten Blickrichtungen und/oder des ermittelten Nah-Durchblickpunktes umfassen, sodass ein Bediener wie ein Optiker eine Korrektur dieser Größen, d.h. der Blickrichtungen und/oder des Nah-Durchblickpunktes, vornehmen kann, zum Beispiel falls er der Meinung ist, dass die zu untersuchende Person bei dem Aufnehmen des Bildes nicht die korrekte Kopfhaltung eingenommen hat. Auf diese Weise können also Ungenauigkeiten, die sich aus einer derarigen nicht korrekten Kopfhaltung ergeben, noch korrigiert werden.

Bei manchen der oben beschriebenen Verfahren wird wie beschrieben die Brillenfassung in dem aufgenommenen Bild identifiziert und die so bestimmte Lage der Brillenfassung zur Bestimmung des Nah-Durchblickpunktes herangezogen. Hierfür trägt die Person bei der Aufnahme des Bildes die Brillenfassung.

Bei anderen Ausführungsformen wird ein virtuelles Modell, insbesondere ein 3D-Modell, des Kopfes der Person verwendet. An das 3D-Modell kann ein Modell einer Brillenfassung angepasst sein.

Das 3D-Modell kann in manchen basierend auf Aufnahmen der selben Kameraeinrichtung, wie sie für zuvor und nachfolgend erläuterte Ausführungsbeispiele verschiedener Erfindungsaspekte verwendet wird, bestimmt werden. Dies kann den Vorteil haben, dass die so bestimmten 3D-Modelle einfacher mit Aufnahmen der Kameraeinrichtung verglichen werden können.

Auf Basis dieser Modelle (Modell des Kopfes und dem angepassten Modell der Brillenfassung) und der wie oben beschrieben ermittelten Blickrichtung kann dann wiederum der Nah-Durchblickpunkt bestimmt werden, da die Lage der Brillenglasebenen und die Augendrehpunkte dem 3D-Modell des Kopfes mit angepasstem Modell der Brillenfassung entnehmbar sind. Die Augendrehpunkte können auch dem 3D-Modell des Kopfes ohne dem 3D-Modell der Brillenfassung entnommen werden.

In manchen Ausführungsbeispielen können die Augendrehpunkte als Metadaten gespeichert werden.

Unter Metadaten sind Daten zu verstehen, welche Informationen über die Merkmale des Modells, jedoch nicht das Modell selbst enthalten. Insbesondere können die Metadaten Zusatzinformationen zu den Modellen liefern und/oder markante Punkte oder Kurven basierend auf dem jeweiligen Modell enthalten, beispielsweise den Augendrehpunkt, der basierend auf dem Modell berechnet werden kann, nicht aber Teil des Modells ist. Beispielsweise kann im Modell die Cornea identifiziert werden und der Augendrehpunkt basierend auf der räumlichen Anordnung der Cornea bestimmt werden. Metadaten sind auch in dem Wikipedia-Artikel "Metadaten", Stand 7. Juni 2018, allgemein erläutert.

Ein Vorteil der Verwendung von Modellen ist, dass schnell zwischen verschiedenen Brillenfassungen gewechselt werden kann, ohne dass erneut eine Bildaufnahme erfolgen muss. Dabei wird angenommen, dass die Blickrichtung beim Blick auf das Nahblickziel unabhängig von der getragenen Brillenfassung ist.

Unter einem Modell, insbesondere 3D-Modell, ist eine Repräsentation, im Falle eines 3D-Modells eine dreidimensionale Repräsentation von realen Objekten zu verstehen, die als Datensatz in einem Speichermedium, beispielsweise einem Speicher eines Computers oder einem Datenträger, vorliegen. Eine solche dreidimensionale Repräsentation kann beispielsweise ein 3D-Netz (englisch "3D mesh"), bestehend aus einem Satz von 3D-Punkten, die auch als Vertices bezeichnet werden, und Verbindungen zwischen den Punkten, die auch als Edges bezeichnet werden. Diese Verbindung bilden im einfachsten Fall ein Dreiecksnetz (englisch triangle mesh). Bei einer derartigen Repräsentation als 3D-Netz wird nur die Oberfläche eines Objekts beschrieben, nicht das Volumen. Das Netz muss nicht notwendigerweise geschlossen sein. Wird also beispielsweise der Kopf in Form eines Netzes beschrieben, so erscheint er wie eine Maske. Näheres zu derartigen 3D-Modellen findet sich in Rau J-Y, Yeh P-C. "A Semi-Automatic Image-Based Close Range 3D Modeling Pipeline Using a Multi-Camera Configuration." Sensors (Basel, Switzerland). 2012; 12 (8):11271-11293. doi:10.3390/s120811271; insbesondere Seite 11289, Abbildung "Fig.16".)

Eine weitere Möglichkeit der Repräsentation eines 3D-Modells ist ein Voxel-Gitter (englisch "voxel grid"), welches eine volumenhafte Repräsentation darstellt. Dabei wird der Raum in kleine Würfel oder Quader eingeteilt, welche als Voxel bezeichnet werden. Für jedes Voxel wird im einfachsten Fall die An- oder Abwesenheit des darzustellenden Objekts in Form eines Binärwerts (1 oder 0) gespeichert. Bei einer Kantenlänge der Voxel von 1 mm und einem Volumen von 300 mm x 300 mm x 300 mm, was ein typisches Volumen für einen Kopf darstellt, erhält man somit insgesamt 27 Millionen derartiger Voxel. Derartige Voxel-Gitter sind beispielsweise in M. Nießner, M. Zollhöfer, S. Izadi, and M. Stamminger. "Real-time 3D reconstruction at scale using voxel hashing". ACM Trans. Graph. 32, 6, Article 169 (November 2013),. DOI: https://doi.org/10.1145/2508363.2508374 beschrieben.

Die Brillenglasebene bezeichnet eine Ebene, welche die Lage eines jeweiligen Brillenglases (das im Allgemeinen gekrümmt ist) annähert. Sie kann insbesondere eine Ebene sein, die eine geringste Abweichung (beispielsweise nach dem Kriterium der kleinsten Quadrate) von dem Fassungsrand des jeweiligen Brillenglases aufweist. Der Fassungsrand kann wie in der EP 17153651.9 beschrieben identifiziert werden. Alternativ kann als Brillenglasebene die Scheibenebene gemäß DIN EN ISO 13666:2012; 17.1 verwendet werden. Die Bestimmung dieser Scheibenebene setzt jedoch wie in der DIN EN ISO 13666:2012 die Kenntnis der Form einer Stützscheibe voraus, während bei der obigen Definition lediglich die Lage des Fassungsrandes benötigt wird.

Die Anpassung des Modells der Brillenfassung an das Modell des Kopfes kann dabei wie in der US 2003/0123026 A1, der US 2002/105530 A1, der US 2016/0327811 A1 oder der europäischen Patentanmeldung Nr. 17173929.5 beschrieben erfolgen.

Bei einer Ausführungsform wird die Änderung der Kopfhaltung beim Blick auf das Nahblickziel relativ zur Kopfhaltung beim Blick in die Ferne bestimmt. Dies kann mittels einem oder mehrerer Bilder wie oben beschrieben erfolgen, wobei in diesem Fall bei der Aufnahme des einen oder der mehreren Bilder von der Person keine Brille getragen werden muss. Das 3D-Modell des Kopfes kann dann entsprechend der Änderung der Kopfhaltung geneigt werden oder im Falle von mehreren Kameras auf Basis der Bilder erstellt werden. Durch Identifizierung einander entsprechender Merkmale in den aufgenommen Bildern kann dann unter Berücksichtigung der bekannten Aufnahmepositionen das Modell erstellt werden (siehe z.B. H. Hirschmuller, "Stereo Processing by Semiglobal Matching and Mutual Information," in IEEE Transactions on Pattern Analysis and Machine Intelligence, vol. 30, no. 2, pp. 328-341, Feb. 2008. doi: 10.1109/TPAMI.2007.1166). Mit der wie oben beschrieben bestimmten Blickrichtung beim Blick auf das Nahblickziel ist dann nach dem virtuellen Anpassen des Modells der Brillenfassung bekannt, wo die Fern- und Nah-Durchblickpunkte liegen, da z.B. damit die relative Lage von Augendrehpunkt und Brillenglasebenen entsprechend den Fassungsrändern aus den Modellen bekannt sind.

Bei einer anderen Ausführungsform werden die Positionen der Pupillen im Raum bei Fernblickrichtung und Nahblickrichtung mit Hilfe von Bildaufnahmen bestimmt. Zusammen mit der Position eines weiteren Gesichtspunktes kann dann das Modell des Kopfes in eine Position entsprechend der Kopfhaltung der Person beim Blick auf das Nahblickziel gebracht werden. Als weiterer Gesichtspunkt kann die Nasenspitze oder ein Punkt an den Ohren herangezogen werden, da die relative Lage dieser Punkte zu den Pupillen sich bei Bewegungen kaum ändert. Ansonsten erfolgt die Bestimmung des Nah-Durchblickpunktes wie oben beschrieben.

Alternativ kann die Veränderung der Kopfhaltung beim Blick auf das Nahblickziel auch nur auf Basis von Seitenbildern des Kopfes, die beim Blick in die Ferne und beim Blick auf das Nahblickziel aufgenommen werden, erfolgen. Durch Identifizieren entsprechender Merkmale in den Seitenbildern (z.B. wie in der obigen Referenz Hirschmüller et al. beschrieben) kann dann die Veränderung der Kopfneigung bestimmt und auf das 3D-Modell übertragen werden.

Seitenbilder des Kopfes sind Bilder, auf welchen der Kopf von der Seite, im Wesentlichen in Richtung einer Schläfenpartie des Kopfes, sichtbar ist. In anderen Worten erfolgt die Bildaufnahme bei Seitenbildern im Wesentlichen in einer Richtung, die einer Verbindungslinie zwischen den Ohren des Kopfes entspricht. In einem Seitenbild ist nur ein Auge der Person sichtbar.

Frontbilder sind demgegenüber Bilder, bei denen die Bildaufnahme im Wesentlichen senkrecht zu einer Ebene, welche durch die Augen und den Mund definiert ist, erfolgt. Hier sind im Regelfall beide Augen, die Nase und der Mund abgebildet.

Somit gibt es verschiedene Möglichkeiten, die Lage des Nah-Durchblickpunktes auf Basis von Modellen von Kopf und Brillenfassung zu bestimmen.

Die Verwendung einer (realen) Brillenfassung bei den Bildaufnahmen kann auch mit der Verwendung von Modellen von Kopf und Brillenfassung kombiniert werden. Hierbei wird der Nah-Durchblickpunkt zunächst wie beschrieben für die reale Brillenfassung bestimmt. Ergebnisse dieser Bestimmung wie Kopfhaltung und Blickrichtung können dann auf die Verwendung der Modelle übertragen werden. Somit können die Ergebnisse für den Nah-Durchblickpunkt auf andere Brillenfassungen mittels Modellen derselben übertragen werden, ohne dass erneut Bilder aufgenommen werden müssen.

Die Position und/oder Orientierung des Nahblickziels kann auch manuell (beispielsweise mit einem Meterstab) bestimmt werden und dann manuell eingegeben werden. Die Position der Pupillen kann auch geschätzt werden.

Gemäß einer weiteren Ausführungsform wird ein Computerprogramm mit einem Programmcode bereitgestellt, welches, wenn es auf einer Recheneinrichtung ausgeführt wird, bewirkt, dass eines der oben beschriebenen Verfahren bereigestellt wird.

Gemäß einem weiteren Aspekt wird eine Vorrichtung zum Bestimmen eines Nah-Durchblickpunktes bereitgestellt, umfassend:

- ein bewegbares Nahblickziel,
- eine Bildaufnahmeeinrichtung zum Aufnehmen eines Bildes eines Kopfes einer Person, während die Person auf das Nahblickziel blickt,
- eine Positionserfassungseinrichtung zum Bestimmen einer Position und/oder Orientierung des Nahblickziels, und

- eine Recheneinrichtung zum Bestimmen des Nah-Durchblickpunktes auf Basis des Bildes und der Position und/oder der Orientierung des Nahblickziels.

Diese Vorrichtung ermöglicht wie das entsprechende oben beschriebene Verfahren eine freie Positionierung des Nahblickziels und somit eine Einnahme einer natürlichen Kopfhaltung bei der Bestimmung des Nah-Durchblickpunktes sowie eine Bestimmung des Nah-Durchblickpunktes ohne Messbügel.

Die Vorrichtung kann zur Durchführung einer der oben beschriebenen Verfahren eingerichtet sein. Insbesondere kann hierzu die Bildaufnahmeeinrichtung wie oben beschrieben zwei oder mehr Kameras umfassen, mit welchen ein Frontbild und ein oder mehrere Seitenbilder gleichzeitig aufnehmbar sind. Auch kann das Nahblickziel die Kameraeinrichtung und/oder Lagesensoren als Positionserfassungseinrichtung umfassen, wie oben beschrieben. Die Positionserfassungseinrichtung kann wie ebenfalls beschrieben mechanische Sensoren oder ein oder mehrere Kameras umfassen. Das ober erwähnte Computerprogramm kann in der Vorrichtung gespeichert sein, um durch die Recheneinrichtung ausgeführt zu werden.

Im Folgenden werden Ausführungsbeipsiele der vorliegenden Erfindung unter Bezugnahme auf die beiliegenden Zeichnungsfiguren näher erläutert. Es zeigen:

Fig. 1 eine Vorrichtung gemäß den Stand der Technikzur Bestimmung eines Fern-Durchblickpunktes, auf Basis derer die Erfindungs implementiert sein kann.,

Fig. 2 eine Draufsicht auf die Vorrichtung der Fig. 1,

Figuren 3A und 3B erläuternde Ansichten zur Bestimmung eines Fern-Durchblickpunktes,

Fig. 4 eine Erweiterung der Vorrichtung der Fig. 1 für eine Bestimmung eines Nah-Durchblickpunktes gemäß einem Ausführungsbeispiel der vorliegenden Erfindung,

Figuren 5A und 5B Diagramme zur Veranschaulichung der Bestimmung von Nah-Durchblickpunkten gemäß Ausführungsbeispielen der Erfindung,

Fig. 6 eine schematische Ansicht eines Nahblickziels gemäß einem Ausführungsbeispiel, und

Fig. 7 ein Flussdiagramm eines Verfahrens gemäß einem Ausführungsbeispiel.

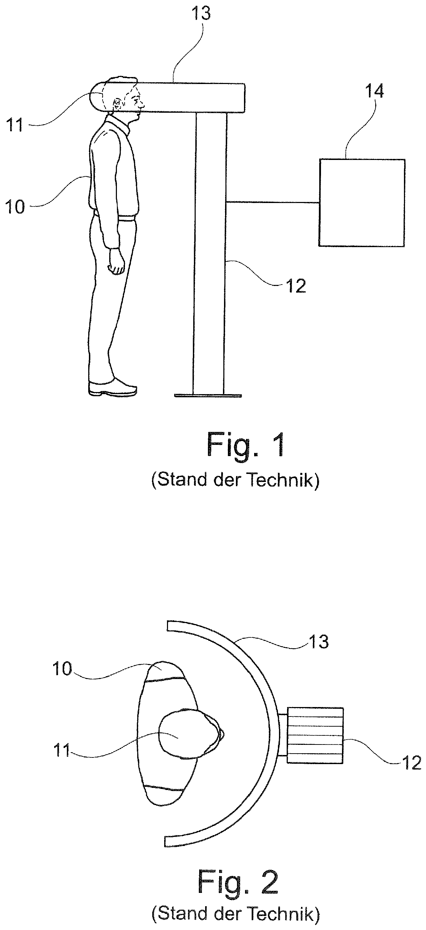

Die Fig. 1 zeigt eine Vorrichtung, wie sie in den bereits erwähnten europäischen Patentanmeldungen 17 153 559.4 und 17 153 556.0 näher beschrieben ist, in einer Seitenansicht. Auf Basis dieser Vorrichtung kann als Erweiterung die vorliegende Erfindung implementiert werden. Die Fig. 2 zeigt eine Draufsicht auf die Vorrichtung der Fig. 1. Die Vorrichtung umfasst eine Kameraeinrichtung 13, welche auf einer Säule 12 angebracht ist. Die Kameraeinrichtung 13 umfasst mehrere Kameras, unter anderem eine Frontkamera zum Aufnehmen eines Frontbildes eines Kopfes 11 einer zu untersuchenden Person 10 sowie Seitenkameras zum Aufnehmen von Seitenbildern des Kopfes 11. In den Figuren 1 und 2 hat die Person 10 eine Kopfhaltung zur Bestimmung eines Fern-Durchblickpunktes, d. h. sie blickt im Wesentlichen horizontal geradeaus auf ein in der Vorrichtung bereitgestelltes Ziel. Eine Recheneinrichtung 14 ist mit der Vorrichtung zur Auswertung der aufgenommenen Bilder verbunden, insbesondere um die Fern-Durchblickpunkte zu bestimmen.

Wie in den oben genannten europäischen Patentanmeldungen dabei näher erläutert, werden im Zuge der Bestimmung des Fern-Blickpunktes eine Lage einer Brillenfassung, welche die Person 10 an dem Kopf 11 trägt, sowie Lagen der Pupillen der Person bestimmt. Die Lage der Brillenfassung oder zumindest von Fassungsrändern hiervon und der Pupillen und/oder Cornea (Hornhaut), kann dabei manuell durch Markieren der Brillenfassung und Pupillen in dargestellten Bildern oder automatisch durch Bildverarbeitungsalgorithmen erfolgen, wie ebenfalls beschrieben. Ist das Auge, beispielsweise die Cornea des Auges, in den Seitenbildern nicht genau zu erkennen, weil es durch Brillenbügel verdeckt wird, kann auch zusätzlich ein Bild des Kopfes ohne Brillenfassung eingeblendet werden, um eine Bestimmung der Position der Pupille und/oder der Cornea zu ermöglichen. Hierbei kann die Bestimmung der Position der Cornea bzw. der Pupille der Person ebenfalls automatisiert vorgenommen werden, wie zuvor für den Fall, dass die Augen nicht durch die Brillenfassung verdeckt sind, beschrieben.

Das Bild des Kopfes ohne Brillenfassung kann hierbei als ein weiteres Seitenbild auf Basis eines 3D-Modells des Kopfes der Person bereitgestellt werden. Dieses 3D-Modell des Kopfes kann auf Basis von Bildaufnahmen der mehreren Kameras der Kameraeinrichtung 13 erstellt werden, also auf Basis mehrerer Bildaufnahmen wie eingangs erläutert. Dieses 3D-Modell wird dabei ohne Brillenfassung erstellt, d.h. die Bildaufnahmen zur Erstellung des 3D-Modells werden von dem Kopf der Person gemacht, ohne dass die Person die Brillenfassung trägt.

Dazu wird das weitere Seitenbild auf Basis des 3D-Modells des Kopfes mit Techniken der Bildsynthese (engl. Rendern) so erzeugt, als würde der Betrachter von dem Ort aus, an dem sich die Kamera (z.B. der Kameraeinrichtung 13) befindet, die das Seitenbild aufnimmt, den Kopf, aus dem das 3D-Modell erzeugt wurde, betrachten. In anderen Worten stimmt die Aufnahmerichtung bei der Aufnahme des Seitenbildes mit der Blickrichtung auf das weitere Seitenbild überein. Unter einer Bildsynthese wird dabei allgemein die Erzeugung einer Grafik aus einem Modell verstanden, siehe z.B. der Wikipedia-Artikel "Bildsynthese".

Das Seitenbild und das weitere Seitenbild werden dann zur Deckung gebracht, d.h. in Größe, Position und Ausrichtung einander angepasst. Hierzu können bestimmte Punkte oder Bildbereiche mit herkömmlichen Verfahren der Bildverarbeitung in dem Seitenbild und dem weiteren Seitenbild identifiziert werden und dann zur Deckung gebracht werden, d.h. diese Punkte oder Regionen werden auf die gleiche Position gebracht. Geeignete Punkte umfassen die Nasenspitze oder die Kinnspitze. Geeignete Bildbereiche umfassen die Stirn oder das Kinn. Eine mögliche Variante hierzu wird nachfolgend genauer beschrieben:

Ausgehend von einer Ausgangsposition des 3D-Modells des Kopfes kann das 3D-Modell des Kopfes durch eine Transformation mit einer Translation und einer Rotation in eine Endposition des Kopfes gebracht werden. Eine solche Transformation des Kopfes wird auch als Ausrichten bezeichnet.

Die Ausgangsposition kann hierbei durch den Koordinatenursprung des 3D-Modells und die Wahl der Koordinatenrichtungen, z.B. kopffeste Achsen vertikal / horizontal seitlich / horizontal frontal vorgegeben sein.

Unter Transformation werden maßerhaltene Koordinatentransformationen, beispielsweise eine Rotation um bis zu drei Raumwinkel und eine Translation entlang von bis zu drei Koordinatenachsen, verstanden.

Parameter T der Translation, also Größe und Richtung der Translation (beispielsweise ausgedrückt durch Verschiebungen entlang von drei Koordinatenachsen) und Parameter R der Rotation, also die Größe der Rotation (beispielsweise ausgedrückt durch Rotationswinkel um die drei Koordinatenachsen) können automatisiert oder manuell bestimmt werden.

Hierzu können sowohl bei automatisierter als auch bei manueller Bestimmung charakteristische Regionen, beispielsweise charakteristische Punkte, Konturen oder Flächen in dem Seitenbild oder auch anderen Bildaufnahmen dem weiteren Seitenbild auf Basis des 3D-Modells oder anderen auf Basis des 3D-Modells erzeugten Bildern der Person identifiziert werden, die sowohl mit als auch ohne Brille erkennbar und idealerweise unbeeinflusst von der Mimik sind. Diese können beispielsweise sein: Ohransatz, Nase, Kontur von Stirn/Nase/Kinn von der Seite betrachtet. Aber auch andere charakteristische Regionen sind möglich. Beim automatischen Ausrichten kann es einem Algorithmus überlassen werden, welche Merkmale selektiert werden. Das Ausrichten kann iterativ erfolgen. Die Translation kann in einem ersten Schritt durch eine Schwerpunktbestimmung erfolgen, beispielsweise indem ein Schwerpunkt von mindestens einem Teil der Koordinaten des 3D-Modells bestimmt wird und ein zweites 3D-Modell einer Person, die eine Brillenfassung trägt, wie zuvor beschrieben, erstellt wird und ebenfalls ein Schwerpunkt für das zweite 3D-Modell bestimmt wird. Die Parameter T der Translation können dann als die vektorielle Differenz der Schwerpunkte vom ersten und zweiten 3D-Modell bestimmt werden, oder diese Parameter T können als Ausgangswert für eine genauere Bestimmung der Parameter T, beispielsweise manuell oder durch weitere Algorithmen, verwendet werden.

Die Parameter R der Rotation können entsprechend durch ein zweites Ausrichten anhand des Seitenbildes und des weiteren Seitenbildes bestimmt werden. Hierbei kann abermals die Lage der charakteristischen Regionen bestimmt werden und hierdurch auf eine Drehung des Kopfes und dessen Seitenneigung geschlossen werden.

In manchen Ausführungsbeispielen, insbesondere wenn das 3D-Modell mit derselben Kameraeinrichtung 13 erstellt wurde wie das Seitenbild, weist das weitere Seitenbild die gleiche Größe wie das Seitenbild auf. Gegebenenfalls kann hierzu die Entfernung und/oder der Bildwinkel der virtuellen Kamera, von der aus das weitere Seitenbild durch Bildsynthese erzeugt wird, zu dem 3D-Modell entsprechend angepasst werden. Somit ist hier keine Skalierung erforderlich.

Bei anderen Ausführungsbeispielen kann die Größe der Darstellung eines aus dem 3D-Modell erzeugten Bildes wie des weiteren Seitenbildes angepasst werden, beispielsweise durch eine Skalierung des 3D-Modells oder hieraus erzeugter Bilder wie dem weiteren Seitenbild.

Die ermittelte Transformation kann auch auf Metadaten des 3D-Modells, beispielsweise den Augendrehpunkten, angewendet werden. Für einen Augendrehpunkt Pi im 3D-Modell gilt dann für die transformierte Position Pi':

$$Pi` = (T+R)Pi.$$

Basierend auf den transformierten Positionen Pi' der Augendrehpunkte können dann die Nah-Durchblickpunkte als Schnittpunkte zwischen an den Augendrehpunkten angesetzten Blickrichtungsvektoren (d.h. der Blickrichtung als Vektor gesehen) und den Brillenglasebenen bestimmt werden.

Dieses zur Deckung bringen ist insbesondere auch dann möglich, wenn beide Augen der Person in dem Seitenbild verdeckt sind, beispielsweise durch Nase und Brillenbügel wie oben erwähnt, indem Punkte oder Bildbereiche wie oben diskutiert verwendet werden, die sowohl in dem Seitenbild als auch in dem weiteren Seitenbild vorhanden sind. Somit kann die Position der Cornea und/oder der Pupille der Person basierend auf dem weiteren Seitenbild bestimmt werden, da diese Position in dem 3D-Modell und somit auch in dem weiteren Seitenbild bekannt ist. Diese Bestimmung kann automatisiert erfolgen.

Die ermittelte Position von Cornea und/oder Pupille kann dann zudem in dem Seitenbild dargestellt werden, so dass eine Bestätigung und/oder Korrektur der ermittelten Position durch den Optiker erfolgen kann. Ebenfalls ist es möglich, dass der Optiker die ermittelte Position verwirft und die Messung wiederholt. Zu diesem Zweck kann wahlweise das gesamte weitere Seitenbild als dem Seitenbild überlagert angezeigt werden, oder es kann nur ein Bereich des weiteren Seitenbildes, beispielsweise der Bereich, in dem die Cornea bzw. die Pupille dargestellt ist, über das Seitenbild gelegt werden. Insbesondere kann für diese Darstellung mindestens ein Teil des Seitenbilds durch einen entsprechenden Teil des weiteren Seitenbildes, der die Pupille und/oder Cornea umfasst, ersetzt werden. Ein entsprechender Teil ist dabei ein Teil, der den gleichen Teil des Kopfes zeigt.

Insbesondere kann für die Darstellung und/oder weitere Verarbeitung, beispielsweise mittels den zuvor beschriebenen automatisierten Verfahren zur Bestimmung der Pupillen- und/oder Corneapositionen mindestens ein Teil der Pixel des Seitenbilds durch Pixel des weiteren Seitenbildes ersetzt werden.

Zudem wird, wie bereits oben erläutert, eine Lage eines Augendrehpunktes abgeschätzt. Nach diesen Messungen ist also eine relative Lage des Augendrehpunktes zu der Brillenfassung sowie eine Form der Brillenfassung bekannt. Die Lage der Brillenfassung bestimmt dabei die Lage der Brillengläser und somit von Ebenen, in denen die Brillengläser liegen (auch als Glasebenen bezeichnet).Mit diesen Daten können in der in den oben genannten europäischen Patentanmeldungen beschriebenen Weise dann Fern-Durchblickpunkte bestimmt werden.

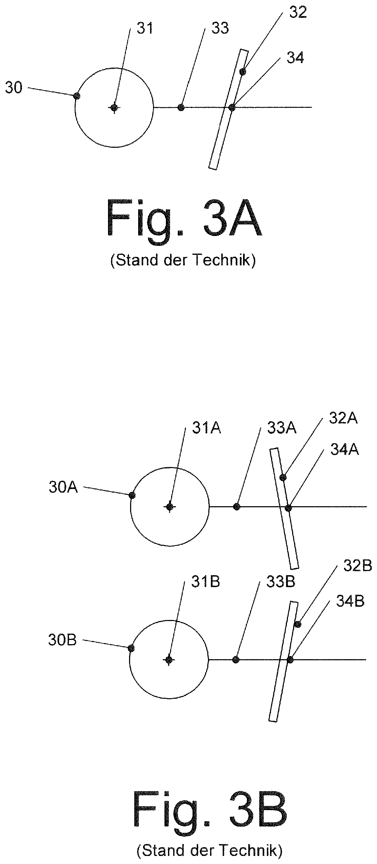

Diese Informationen werden dann bei dem beschriebenen Ausführungsbeispiel zur Bestimmung eines oder beider Nah-Durchblickpunkte verwendet. Zudem können wie in den europäischen Patentanmeldungen beschrieben Fern-Durckblickpunkte bestimmt werden. Dies ist in den Figuren 3A und 3B schematisch dargestellt. Die Figur 3A zeigt eine Lage eines Fern-Durchblickpunktes 34 für eine Blickrichtung 33 ausgehend von einem Augendrehpunkt 31 eines Auges 30 als Schnittpunkt der Blickrichtung 33 mit einem Brillenglas 32 in Seitenansicht. Die Fig. 3B zeigt eine Draufsicht für beide Augen, wobei die Bezugszeichen für das linke Auge jeweils ein angehängtes A aufweisen und die Bezugszeichen für das rechte Auge ein angehängtes B, und ansonsten den Bezugszeichen der Seitenansicht der Fig. 3A entsprechen.

Die Lage der Brillengläser 32 ergibt sich dabei aus den in den Bildaufnahmen identifizierten Brillenfassungen, insbesondere den Fassungsrändern hiervon.

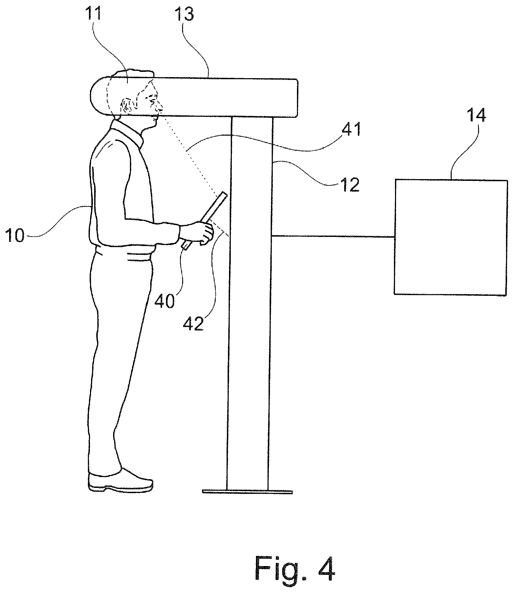

Die Fig. 4 zeigt eine Seitenansicht der Vorrichtung der Fig. 1, welche zu einer erfindungsgemäßen Bestimmung eines Nah-Durchblickpunktes erweitert ist. Hier wird der Person 10 zusätzlich zu der Vorrichtung der Fig. 1 ein Nahblickziel 40 bereitgestellt, welches einen zu lesenden Text aufweist und auch als Lesetarget bezeichnet wird. Das Nahblickziel 40 ist dabei durch die Person 10 beweglich, um es in eine Position zu bringen, die einer angenehmen Lesehaltung entspricht. Das Bezugszeichen 41 bezeichnet die Blickrichtung der Person 10 beim Lesen des Textes. Bei dem Ausführungsbeispiel der Fig. 4 ist das Nahblickziel 40 durch eine bewegliche Verbindung 42, die einen oder mehrere Sensoren umfasst, mit der Säule 12 verbunden. Mittels dieser Sensoren wird die Position des Nahblickziels 40 erfasst. Hierbei kann der mindestens eine Sensor eingerichtet sein, ein Signal basierend auf der Position der beweglichen Verbindung 42 an eine Recheneinrichtung 14 bereitzustellen. Zudem werden durch die Kameraeinrichtung 13 in der in Fig. 4 dargestellten Position Bildaufnahmen des Kopfes 11 vorgenommen. In diesen Bildaufnahmen wird wiederum die Brillenfassung identifiziert. Dies kann gemeinsam mit der Identifizierung der Brillenfassung bei den Aufnahmen der Fig. 1 oder nach dieser erfolgen, wobei im letzteren Fall die in Fig. 1 bestimmte Brillenfassung als Basis verwendet wird. Als Randbedingungen können hier beispielsweise Modelle für Brillenfassungen dienen, wie sie in der europäischen Patentanmeldung 17 153 538.8 beschrieben sind.

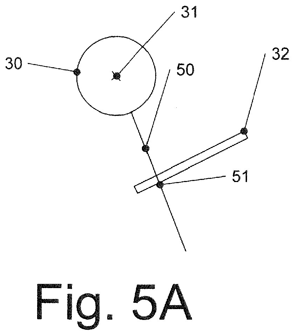

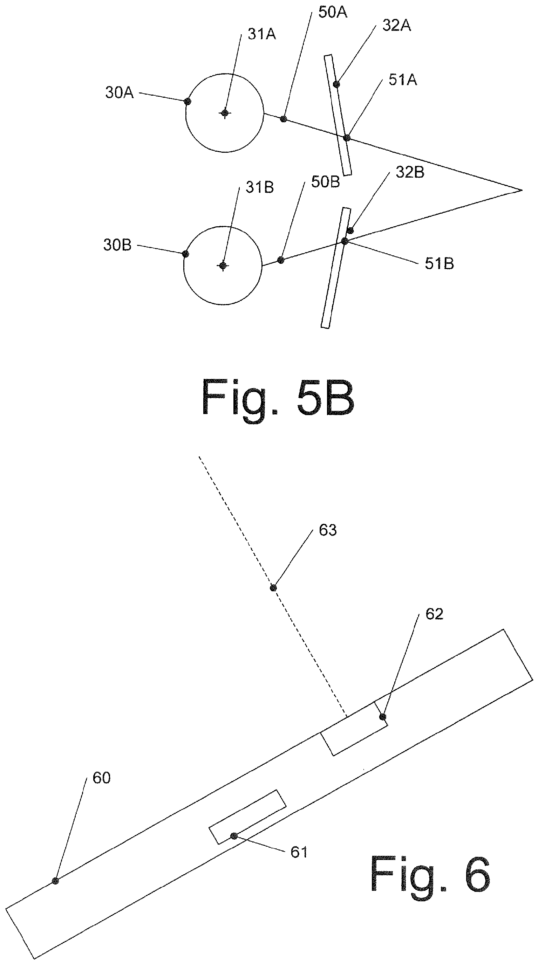

Die Recheneinrichtung 14 ist dann in Ergänzung zu der herkömmlichen Vorrichtung der Fig. 1 noch programmiert, Nah-Durchblickpunkte für beide Augen zu bestimmen. Hierbei wird die bei der Bestimmung der Fern-Durchblickpunkte in der Position der Fig. 1 ermittelte relative Lage des Augendrehpunktes zu der Fassung auf die nun bestimmte Lage der Brillenfassung übertragen und als Blickrichtung die Differenz der Position dieses Augendrehpunktes zu der Position des Nahblickziels 40 verwendet. So wird die Blickrichtung 41 bestimmt. Die Blickrichtung 41 ausgehend vom Augendrehpunkt schneidet die Brillengläser, deren Lagen durch die Lage der Brillenfassung definiert sind, dann in den Nah-Durchblickpunkten. Dies ist in den Figuren 5A und 5B schematisch dargestellt. Die Figuren 5A und 5B zeigen dabei Ansichten entsprechend den Figuren 3A und 3B für die Bestimmung von Nah-Durchblickpunkten 51 in Fig. 5A bzw. 51A, 51B in Fig. 5B. Die Blickrichtung 50 (50A, 50B für linkes und rechtes Auge) schneiden die Brillengläser 32A, 32B in den Nah-Durchblickpunkten 51A, 51B, welche sich wie aus einem Vergleich der Figuren 5A, 5B mit den Figuren 3A, 3B ersichtlich von den Fern-Durchblickpunkten unterscheiden.

Wie bereits eingangs erläutert kann alternativ zu der Positionsbestimmung des Nahblickziels 40 und die Bildaufnahme durch die Kameraeinrichtung 13 eine Bildaufnahme durch das Nahblickziel und eine Orientierungsbestimmung des Nahblickziels erfolgen. Ein entsprechendes Nahblickziel ist in Fig. 6 dargestellt.

Die Fig. 6 zeigt ein Nahblickziel 60, welches über eine eingebaute Kamera 62 verfügt. Die Kamera 62 weist eine Symmetrieachse 63 auf, welche der optischen Achse der Kamera 62 entspricht. Des Weiteren weist das Nahblickziel 60 einen Neigungssensor 61 auf. Das Nahblickziel 60 ist dabei ein Tablet-Computer oder Smartphone, welche üblicherweise über Neigungssensoren sowie eingebaute Kameras verfügen. Bei der Ausführungsform der Fig. 6 wird mittels der Kamera 62 ein Bild des Kopfes der Person aufgenommen, während sie das Nahblickziel 60 betrachtet. Aus der Orientierung des Nahblickziels 60 wie durch den Neigungssensor 61 bestimmt und somit der Orientierung der Symmetrieachse 63, den Eigenschaften der Kamera 62 (Bildwinkel und Auflösung) sowie dem aufgenommenen Bild kann dann wie beschrieben die Blickrichtung der Person bestimmt werden, und auf Basis der Blickrichtung dann wie oben beschrieben die Nahdurchblickpunkte.

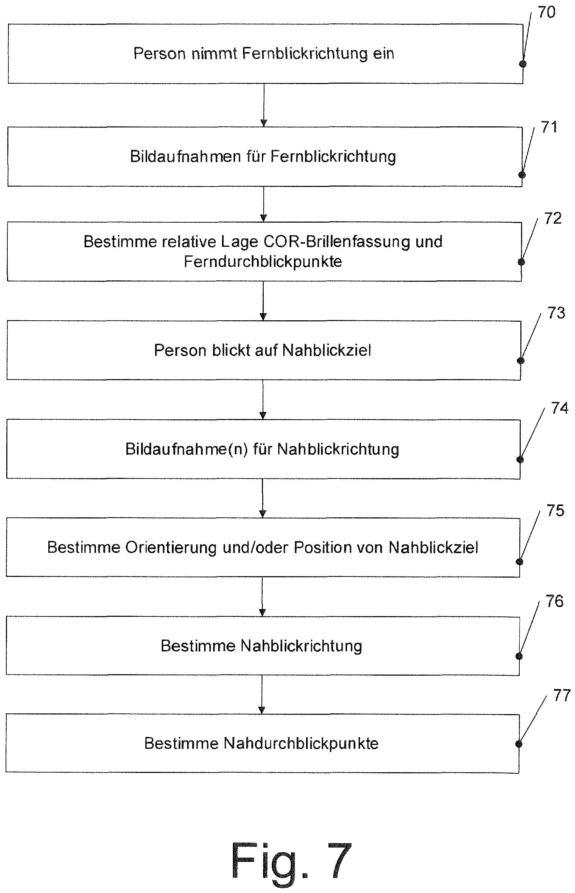

Die Fig. 7 zeigt ein Flussdiagramm eines Verfahrens gemäß einem Ausführungsbeispiel. Das Verfahren der Fig. 7 wird zur besseren Veranschaulichung unter Bezugnahme auf die in den Figuren 1 bis 6 dargestellten Vorrichtungen erläutert.

In Schritt 70 nimmt eine zu untersuchende Person eine Fernblickrichtung ein, beispielsweise die in den Figuren 1 und 2 dargestellte Position, bei welchen der Benutzer gerade horizontal nach vorne blickt. In Schritt 71 erfolgen dann Bildaufnahmen für die Fernblickrichtung, im Falle der Figuren 1 und 2 mittels der Kameraeinrichtung 13. Aus diesen Bildaufnahmen wird wie bereits beschrieben in Schritt 72 die Lage einer Brillenfassung, die Lage von Pupillen und daraus die relative Lage des Augendrehpunktes (COR; Center of Rotation) zu der Brillenfassung bestimmt. Zudem werden in Schritt 72 Fern-Durchblickpunkte wie in den Figuren 3A und 3B erläutert bestimmt.

In Schritt 73 blickt die Person dann auf ein Nahblickziel, wie dies in der Fig. 4 für das Nahblickziel 40 gezeigt ist, oder auf das Nahblickziel 60 der Fig. 6. In Schritt 74 erfolgen dann eine oder mehrere Bildaufnahmen für die Nahblickrichtung, beispielsweise mehrere Bildaufnahmen mittels der Kameraeinrichtung 13 der Fig. 4 oder eine Bildaufnahme mit der Kamera 62 des Nahblickziels 60 der Fig. 6. In Schritt 75 werden Orientierung (beispielsweise mit dem Lagesensor 61) und/oder Position (beispielsweise mit den Sensoren der Verbindung 42 der Fig. 4) des Nahblickziels bestimmt. Aus den so ermittelten Daten wird wie beschrieben in Schritt 76 die Nahblickrichtung bestimmt. Optional kann in Schritt 76 zusätzlich eine Korrektur der Nahblickrichtung durch einen Benutzer wie einen Optiker erfolgen. Diese Korrektur kann in verschiedenen Richtungen erfolgen, beispielsweise falls die Person zum Zeitpunkt der Bildaufnahme keine natürliche Kopfhaltung eingenommen hat. In Schritt 77 werden dann Nah-Durchblickpunkte bestimmt, indem die Nahblickrichtung ausgehend von dem Augendrehpunkt mit den Brillengläsern geschnitten wird, wie dies in den Figuren 5A und 5B gezeigt ist.

Die Kameraeinrichtung 13 weist dabei intrinsisch und extrinsisch kalibrierte Kameras auf, d. h. die Lage der Kameras zueinander sowie deren Richtungen und Eigenschaften sind bekannt, und beispielsweise Bildfeldverzeichnungen können herausgerechnet werden.

**Patentansprüche**

1. Verfahren zum Bestimmen eines Nah-Durchblickpunktes (51), umfassend:

   Aufnehmen eines Frontbildes und eines ersten Seitenbildes eines Kopfes einer Person, während die Person eine Brillenfassung trägt und auf ein Nahblickziel (40; 60) blickt, wobei das Nahblickziel (40; 60) beweglich ist,
   Bestimmen einer Position und Orientierung des Nahblickziels (40; 60), und
   Bestimmen einer Lage der Brillenfassung basierend auf dem Frontbild und dem ersten Seitenbild,
   Bestimmen einer Blickrichtung der Person beim Blick auf das Nahblickziel, und
   Bestimmen des Nah-Durchblickpunktes (51) der Brillenfassung auf Basis der Blickrichtung und der Lage der Brillenfassung,
   **dadurch gekennzeichnet, dass**
   das Bestimmen der Blickrichtung umfasst:

   Erzeugen eines zweiten Seitenbilds des Kopfes basierend auf einem 3D-Modell des Kopfes,
   Bestimmen der Position einer Pupille und/oder einer Cornea eines Auges der Person basierend auf dem ersten Seitenbild und dem zweiten Seitenbild,
   Berechnen der Blickrichtung als Differenz zwischen der Position des Nahblickziels (40; 60) und der Position der Pupille oder der Cornea.

2. Verfahren nach Anspruch 1, wobei das zweite Seitenbild so erzeugt wird, als würde ein Betrachter von dem Ort aus, von dem eine Kamera das erste Seitenbild aufgenommen hat, den Kopf betrachten, aus dem das 3D-Modell erzeugt wurde.

3. Verfahren nach Anspruch 1 oder 2, **dadurch gekennzeichnet, dass** das Bestimmen der Position der Pupille und/oder der Cornea basierend auf dem ersten Seitenbild und dem zweiten Seitenbild ein zur Deckung bringen des ersten Seitenbildes mit dem zweiten Seitenbild umfasst.

4. Verfahren nach einem der Ansprüche 1-3, **dadurch gekennzeichnet, dass** das Bestimmen der Position der Pupille und/oder der Cornea basierend auf dem ersten Seitenbild und dem zweiten Seitenbild ein Ersetzen eines Teils des ersten Seitenbildes durch einen entsprechenden Teil des zweiten Seitenbildes, der die Pupille und/oder Cornea beinhaltet, umfasst.

5. Verfahren nach einem der Ansprüche 1-4, weiter umfassend:

Bestimmen einer relativen Lage einer Brillenfassung zu einem Augendrehpunkt der Person, wobei der Nah-Durchblickpunkt durch Ermitteln eines Schnittpunktes einer Linie in Blickrichtung ausgehend von dem Augendrehpunkt mit einer durch die Lage der Brillenfassung definierten Lage einer Brillenglasebene (32) bestimmt wird.

6. Verfahren nach Anspruch 5, wobei das Bestimmen der relativen Lage der Brillenfassung zu dem Augendrehpunkt auf Basis weiterer Bildaufnahmen erfolgt.

7. Verfahren nach Anspruch 6, weiter umfassend Bestimmen eines Fern-Durchblickpunktes auf Basis der weiteren Bildaufnahmen.

8. Verfahren nach Anspruch 6 oder 7, weiter umfassend ein Bestimmen der Lage der Brillenfassung auf Basis der weiteren Bildaufnahmen.

9. Verfahren nach Anspruch 8, weiter umfassend ein Bestimmen einer Lage der Brillenfassung in dem aufgenommen Bild, wobei das Bestimmen des Nah-Durchblickpunktes auf Basis der Lage der Brillenfassung in dem aufgenommenen Bild erfolgt.

10. Verfahren nach Anspruch 9, wobei das Bestimmen der Lage der Brillenfassung in dem aufgenommenen Bild auf Basis des Bestimmens der Lage der Brillenfassung in den weiteren Bildaufnahmen oder gleichzeitig mit dem Bestimmen der Lage der Brillenfassung in den weiteren Bildaufnahmen erfolgt.

11. Verfahren nach einem der Ansprüche 5-10, wobei das Verfahren ein Korrigieren der bestimmten Blickrichtung und/oder ein Korrigieren des bestimmten Nah-Durchblickpunktes umfasst.

12. Computerprogramm mit einem Programmcode, welcher, wenn er auf einem Prozessor ausgeführt wird, bewirkt, dass das Verfahren nach einem der Ansprüche 1-11 durchgeführt wird.

13. Vorrichtung zum Bestimmen eines Nah-Durchblickpunktes (51), umfassend:

    ein Nahblickziel (40; 60), wobei das Nahblickziel (40; 60) beweglich ist,
    eine Bildaufnahmeeinrichtung (13; 62) zum Aufnehmen eines Frontbildes und eines ersten Seitenbildes eines Kopfes einer Person, während die Person auf das Nahblickziel blickt und eine Brillenfassung trägt,
    eine Positionserfassungseinrichtung (42; 61) zum Bestimmen einer Position und Orientierung des Nahblickziels,
    eine Recheneinrichtung (14), die eingerichtet ist zum:

    Bestimmen einer Lage der Brillenfassung basierend auf dem Frontbild und dem ersten Seitenbild, und
    Bestimmen einer Blickrichtung der Person beim Blick auf das Nahblickziel, und
    Bestimmen des Nah-Durchblickpunktes (51) der Brillenfassung auf Basis der Blickrichtung und der Lage der Brillenfassung,
    **dadurch gekennzeichnet, dass**
    die Recheneinrichtung (14) zum Bestimmen der Blickrichtung eingerichtet ist zum:

    Erzeugen eines zweiten Seitenbilds des Kopfes basierend auf einem 3D-Modell des Kopfes,
    Bestimmen der Position einer Pupille und/oder einer Cornea eines Auges der Person basierend auf dem ersten Seitenbild und dem zweiten Seitenbild,
    Berechnen der Blickrichtung als Differenz zwischen der Position des Nahblickziels (40; 60) und der Position der Pupille oder der Cornea.

14. Vorrichtung nach Anspruch 13, wobei das zweite Seitenbild so erzeugt wird, als würde ein Betrachter von dem Ort aus, von dem eine Kamera das erste Seitenbild aufgenommen hat, den Kopf betrachten, aus dem das 3D-Modell erzeugt wurde.

15. Vorrichtung nach Anspruch 13 oder 14, **dadurch gekennzeichnet, dass** die Vorrichtung zur Durchführung des Verfahrens nach einem der Ansprüche 1-11 eingerichtet ist.

## Claims

1. Method for determining a near visual point (51), comprising:

    recording a front image and a first side image of the head of a person while the person wears a spectacle frame and gazes at a near vision target (40; 60), wherein the near vision target (40; 60) is movable,
    determining a position and orientation of the near vision target (40; 60), and
    determining a relative position of the spectacle frame on the basis of the front image and the first side image,
    determining a viewing direction of the person when gazing at the near vision target and
    determining the near visual point (51) of the spectacle frame on the basis of the viewing direction and the relative position of the spectacle frame,
    **characterized in that**

determining the viewing direction comprises:

generating a second side image of the head on the basis of a 3D model of the head, determining the position of a pupil and/or a cornea of an eye of the person on the basis of the first side image and the second side image,

calculating the viewing direction as the difference between the position of the near vision target (40; 60) and the position of the pupil or the cornea.

2. Method according to Claim 1, wherein the second side image is generated in a manner as if an observer would observe the head from which the 3D model was generated from a location from where a camera has recorded the first side image.

3. Method according to Claim 1 or 2, **characterized in that** determining the position of the pupil and/or the cornea on the basis of the first side image and the second side image comprises an aligning of the first side image with the second side image.

4. Method according to any one of Claims 1-3, **characterized in that** determining the position of the pupil and/or the cornea on the basis of the first side image and the second side image comprises a replacement of part of the first side image with a corresponding part of the second side image which contains the pupil and/or cornea.

5. Method according to any one of Claims 1-4, further comprising:

determining a relative position of a spectacle frame with respect to an eye centre of rotation of the person,

wherein the near visual point is determined by ascertaining a point of intersection of a line emanating from the eye centre of rotation in the viewing direction with a relative position of a spectacle lens plane (32) which is defined by the relative position of the spectacle frame.

6. Method according to Claim 5, wherein the relative position of the spectacle frame with respect to the eye centre of rotation is determined on the basis of further image recordings.

7. Method according to Claim 6, further comprising a determination of a far visual point on the basis of the further image recordings.

8. Method according to Claim 6 or 7, further comprising a determination of the relative position of the spectacle frame on the basis of the further image recordings.

9. Method according to Claim 8, further comprising a determination of a relative position of the spectacle frame in the recorded image, the near visual point being determined on the basis of the relative position of the spectacle frame in the recorded image.

10. Method according to Claim 9, wherein the relative position of the spectacle frame in the recorded image is determined on the basis of the determination of the relative position of the spectacle frame in the further image recordings or at the same time as the determination of the relative position of the spectacle frame in the further image recordings.

11. Method according to any one of Claims 5-10, wherein the method comprises a correction of the determined viewing direction and/or a correction of the determined near visual point.

12. Computer program comprising program code which, when executed on a processor, causes the method according to any one of Claims 1-11 to be carried out.

13. Apparatus for determining a near visual point (51), comprising:

a near vision target (40; 60), wherein the near vision target (40; 60) is movable, an image recording device (13; 62) for recording a front image and a first side image of the head of a person while the person gazes at the near vision target and wears a spectacle frame, a position capturing device (42; 61) for determining a position and orientation of the near vision target, a computing device (14) which is set up to:

determine a relative position of the spectacle frame on the basis of the front image and the first side image and determining a viewing direction of the person when gazing at the near vision target and determining the near visual point (51) of the spectacle frame on the basis of the viewing direction and the relative position of the spectacle frame,

**characterized in that** the computing device (14) for determining the viewing direction is set up to:

generate a second side image of the head on the basis of a 3D model of the head, determine the position of a pupil and/or a cornea of an eye of the person on the basis of the first side image and the second side image,

calculate the viewing direction as a difference between the position of the near vision target (40; 60) and the position of the pupil or the cornea.

14. Apparatus according to Claim 13, wherein the second side image is generated in a manner as if an observer would observe the head from which the 3D model was generated from a location from where a camera has recorded the first side image.

15. Apparatus according to Claim 13 or 14, **characterized in that** the apparatus is set up to carry out the method according to any one of Claims 1-11.

**Revendications**

1. Procédé de détermination d'un point de vue proche (51), le procédé comprenant les étapes suivantes :

   - capturer une image de face et une première image de côté d'une tête d'une personne alors que la personne porte une monture de lunettes et regarde une cible de vision (40 ; 60), la cible de vision proche (40 ; 60) étant mobile,
   - déterminer une position et une orientation de la cible de vision proche (40 ; 60), et
   - déterminer une position de la monture de lunettes sur la base de l'image de face et de le première image de côté,
   - déterminer une direction du regard de la personne lorsqu'elle regarde la cible de vision proche, et
   - détermination du point de vue proche (51) de la monture de lunettes sur la base de la direction du regard et de la position de la monture de lunettes, **caractérisé en ce que**
   la détermination de la direction du regard comprend les étapes suivantes :

      générer une deuxième image de côté de la tête à partir d'un modèle 3D de la tête,
      déterminer la position d'une pupille et/ou d'une cornée d'un œil de la personne sur la base de la première image de côté et de la deuxième image de côté,
      calculer la direction du regard en tant que différence entre la position de la cible de vision proche (40 ; 60) et de la position de la pupille ou de la cornée.

2. Procédé selon la revendication 1, la deuxième image de côté étant générée comme si un observateur regardait la tête, à partir de laquelle le modèle 3D a été généré, depuis l'emplacement à partir duquel une caméra a capturé la première image de côté.

3. Procédé selon la revendication 1 ou 2, **caractérisé en ce que** la détermination de la position de la pupille et/ou de la cornée sur la base de la première image de côté et de la deuxième image de côté comprend la mise en coïncidence de la première image de côté avec la deuxième image de côté.

4. Procédé selon l'une des revendications 1 à 3, **caractérisé en ce que** la détermination de la position de la pupille et/ou de la cornée sur la base de la première image de côté et de la deuxième image de côté comprend le remplacement d'une partie de la première image de côté par une partie correspondante de la deuxième image de côté qui contient la pupille et/ou la cornée.

5. Procédé selon l'une des revendications 1 à 4, comprenant en outre les étapes suivantes :

   déterminer une position relative d'une monture de lunettes par rapport à un point de pivotement oculaire de la personne,
   le point de vue proche étant déterminé par détermination d'un point d'intersection d'une ligne dans la direction du regard à partir du centre de rotation oculaire avec une position d'un plan de verre de lunettes (32) qui est défini par la position de la monture de lunettes.

6. Procédé selon la revendication 5, la détermination de la position relative de la monture de lunettes par rapport au point de pivotement oculaire étant effectuée sur la base d'autres captures d'images.

7. Procédé selon la revendication 6, comprenant en outre la détermination d'un point de vue éloigné sur la base des autres captures d'images.

8. Procédé selon la revendication 6 ou 7, comprenant en outre la détermination de la position de la monture de lunettes sur la base des autres captures d'images.

9. Procédé selon la revendication 8, comprenant en outre la détermination d'une position de la monture de lunettes dans l'image capturée, la détermination du point de vue proche étant basée sur la position de la monture de lunettes dans l'image capturée.

10. Procédé selon la revendication 9, la détermination de la position de la monture de lunettes dans l'image capturée est effectuée sur la base de la détermination de la position de la monture de lunettes dans les autres captures d'images ou simultanément à la détermination de la position de la monture de lunettes dans les autres captures d'images.

**11.** Procédé selon l'une des revendications 5 à 10, le procédé comprenant la correction de la direction du regard déterminée et/ou la correction du point de vue proche déterminé.

**12.** Logiciel comprenant un code de programme qui, lorsqu'il est exécuté sur un processeur, déclenche l'exécution du procédé selon l'une des revendications 1 à 11.

**13.** Dispositif de détermination d'un point de vue proche (51), le dispositif comprenant :

une cible de vision proche (40 ; 60), la cible de vision proche (40 ; 60) étant mobile,
un moyen de capture d'image (13 ; 62) destiné à capturer une image de face et une première image de côté d'une tête d'une personne pendant que la personne regarde la cible de vision proche et porte une monture de lunettes,
un moyen de détection de position (42 ; 61) destiné à déterminer une position et une orientation de la cible de vision proche,
un moyen informatique (14) qui est conçu pour :

déterminer une position de la monture de lunettes sur la base de l'image de face et de le première image de côté, et
déterminer une direction du regard de la personne lorsqu'elle regarde la cible de vision proche et
déterminer le point de vue proche (51) de la monture de lunettes sur la base de la direction du regard et de la position de la monture de lunettes,
**caractérisée en ce que**
le moyen informatique (14) destiné à déterminer la direction du regard est conçu pour :

générer une deuxieme image de côté de la tête sur la base d'un modèle 3D de la tête,
déterminer la position d'une pupille et/ou d'une cornée d'un œil de la personne sur la base de la première image de côté et de la deuxième image de côté,
calculer la direction du regard en tant que différence entre la position de la cible de vision proche (40 ; 60) et la position de la pupille ou de la cornée.

**14.** Dispositif selon la revendication 13, la deuxième image de côté étant générée comme si un observateur regardait la tête, à partir de laquelle le modèle 3D a été généré, depuis l'emplacement à partir duquel une caméra a capturé la première image de côté.

**15.** Dispositif selon la revendication 13 ou 14, **caractérisé en ce que** le dispositif est conçu pour mettre en œuvre le procédé selon l'une des revendications 1 à 11.

Fig. 1
(Stand der Technik)

Fig. 2
(Stand der Technik)

# Fig. 3A
(Stand der Technik)

# Fig. 3B
(Stand der Technik)

Fig. 4

Fig. 5A

30A — 31A 50A 32A 51A

30B — 31B 50B 32B 51B

# Fig. 5B

63

62

60 61

# Fig. 6

Person nimmt Fernblickrichtung ein — 70

Bildaufnahmen für Fernblickrichtung — 71

Bestimme relative Lage COR-Brillenfassung und Ferndurchblickpunkte — 72

Person blickt auf Nahblickziel — 73

Bildaufnahme(n) für Nahblickrichtung — 74

Bestimme Orientierung und/oder Position von Nahblickziel — 75

Bestimme Nahblickrichtung — 76

Bestimme Nahdurchblickpunkte — 77

# Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 2005342186 A
- US 2014009737 A1
- US 2010149486 A1
- US 2003123026 A1
- DE 10300188 A1
- EP 2913704 A1
- WO 2014061294 A1
- FR 3021205 A1
- WO 102011009646 A1
- US 20020105530 A1
- EP 2963482 A1
- US 20140293219 A1
- EP 3270099 A1
- EP 1038495 A2
- US 2016011437 A1
- DE 102011009646 A1

- WO 2017064060 A1
- DE 102014200637 A1
- DE 102005003699 A1
- JP 2003329541 A
- EP 17153559 A
- EP 17153556 A
- EP 171535594 A
- EP 17153651
- US 6944320 B2
- US 7149330 B2
- US 20030123026 A1
- US 2002105530 A1
- US 20160327811 A1
- EP 17173929
- EP 17153538 A

**In der Beschreibung aufgeführte Nicht-Patentliteratur**

- **T. DEDEK.** Entwurf und Implementierung einer Objektverfolgung unter Verwendung einer Smart-Camera mit PTZ-Funktionalität. *Bachelorarbeit,* 2009, https://www.ipi.uni-hannover.de/fileadmin/institut/pdf/Abschlussarbeiten/Bachelorarbeit_Dedek_2009.pdf
- **BENNETT, A. ; RABBETTS, R.** Clinical Visual Optics. 1998, S143, , 144
- **RAU J-Y ; YEH P-C.** A Semi-Automatic Image-Based Close Range 3D Modeling Pipeline Using a Multi-Camera Configuration. *Sensors,* 2012, vol. 12 (8), 11271-11293

- **M. NIEßNER ; M. ZOLLHÖFER ; S. IZADI ; M. STAMMINGER.** Real-time 3D reconstruction at scale using voxel hashing. *ACM Trans. Graph.,* November 2013, vol. 32, 6
- **H. HIRSCHMULLER.** Stereo Processing by Semiglobal Matching and Mutual Information. *IEEE Transactions on Pattern Analysis and Machine Intelligence,* Februar 2008, vol. 30 (2), 328-341